# EUROPEAN PATENT APPLICATION

(11) **EP 4 324 400 A1**
(43) Date of publication of application: **21.02.2024**
(21) Application number: 22190906.2
(22) Date of filing: 18.08.2022
(51) Int. Cl.: A61B 5/25, A61B 5/00

(54) **SIGNAL ACQUISITION DEVICE**

(71) Applicant: Koninklijke Philips N.V., 5656 AG Eindhoven (NL)
(72) Inventor: TE VELDE, Mart, Eindhoven (NL); HENDRICKX, Michaël, Eindhoven (NL); MEFTAH, Mohammed, 5656AG Eindhoven (NL)
(74) Representative: Philips Intellectual Property & Standards

(57) **Abstract**

A signal acquisition device (10) comprises an electrode (40) for acquiring a signal from a subject, and an amplifier (20) configured to amplify or buffer the signal from the electrode, wherein the signal acquisition device comprises a cavity (60) housing the amplifier. In accordance with the invention, the electrode comprises the cavity housing the amplifier. The cavity within the electrode provides mechanical and electrostatic shielding to the amplifier.

## Description

### FIELD OF THE INVENTION

The invention relates to a signal acquisition device used for taking electrophysiological measurements.

### BACKGROUND OF THE INVENTION

Signal acquisition devices comprising electrodes can be used for electrophysiological measurements, which involve measurements of voltage changes or electric current in biological tissues. These measurements can be used for monitoring biometric parameters of a subject or for diagnostic purposes, e.g., based on electrocardiography (ECG) or electroencephalography (EEG). Another example is for pregnancy monitoring based on electrical-cardiotocography (eCTG), e.g. determining fetal heart rate (FHR), maternal heart rate (MHR) and uterine activity (UA).

The electrical signals to be detected by such signal acquisition devices are small, e.g. for determining fetal heart rate they are in the order of 10-30 µV. Therefore, it is important to exclude electromagnetic interference (EMI) influencing the electrical signals measured by the signal acquisition devices. Electromagnetic interference may relate to interference from external sources, e.g., electrostatic and electromagnetic coupling between the circuitry and 50 or 60 Hz power lines, fluorescent lights, video monitors, telecom transmissions and noise associated with mechanical vibrations. A part of the signal acquisition devices vulnerable to electromagnetic interference is the conductor transferring the signal acquired by the electrode to a module for processing the acquired signal.

One solution to protect the vulnerable conductor from electromagnetic interference is to passively shield it by surrounding it with a conductive material that is connected to a low-impedance constant voltage source (e.g., device ground). A problem that may arise with this solution is that in case there is a change in the distance between the electrode and the passive shield, e.g. due to bending or vibration of the conductive material with respect to the low-impedance (passive) shield, electrical noise is created as a response. This response is more commonly called the "microphonic effect" and the generated electrical noise may deteriorate the bioelectric signal measured by the signal acquisition device.

Alternatively, an amplifier can be used to amplify the acquired electrical signal, as the amplified signal is less susceptible to electromagnetic interference. The vulnerable conductor may then be actively shielded, by surrounding it with a conductive material that is connected to the output of the amplifier. In order to reduce the effects of electromagnetic interference on the acquired electrical signal, i.e., the signal to be used as input to the amplifier, the amplifier is preferably placed close to the vulnerable conductor to shorten its length. As a result, the amplifier needs to be close to the electrode, thus also close to the skin of the subject. As the amplifier is close to the skin of the subject it is vulnerable and needs protection from electrostatic loads, e.g. caused by friction, and mechanical loads, such as knocks and shear stresses. Furthermore, due to safety reasons, the amplifier should also not come in contact with the skin of the subject.

### SUMMARY OF THE INVENTION

It is, inter alia, an object of the invention to provide a solution for a signal acquisition device comprising an electrode and an amplifier, for protecting the amplifier from electrostatic and mechanical loads.

The invention is defined by the independent claims. Advantageous embodiments are defined in the dependent claims.

A first aspect of the invention provides a signal acquisition device comprising:
an electrode for acquiring a signal from a subject, and
an amplifier configured to amplify or buffer the signal from the electrode to produce an output signal at an output of the signal acquisition device, wherein the signal acquisition device comprises a cavity housing the amplifier, characterized in that:
   the electrode comprises the cavity housing the amplifier.

An advantage of embodiments of the proposed solution is that the electrode, having a cavity wherein the amplifier is housed, protects the amplifier from both mechanical and electrostatic loads.

Furthermore, the electrode may also at least partially house a conductor that connects the output of the electrode with the input of the amplifier. By also housing the conductor in the electrode, the susceptibility of the conductor to electromagnetic interference reduces significantly. In case the conductor was placed outside the electrode, it would act like an antenna being more susceptible to pick up electromagnetic interference. In such a case, protection of the conductor against electromagnetic interference is preferably provided, e.g., by passive or active shielding.

The proposed solution provides a compact signal acquisition device, having fewer components and less complexity than known implementations, i.e., without requiring an extra element such as a casing or using potting compounds for protecting the amplifier from electrostatic or mechanical loads. Also, the proposed solution provides a more robust solution, wherein the components are better mechanically integrated and allowing better miniaturization than known implementations.

The signal acquisition device may comprise a conductive element configured for carrying a controlled voltage, wherein the conductive element is positioned across an opening of the cavity. This solution results to a shielding of the conductor connecting the output of the electrode with the input of the amplifier from electromagnetic interference. This shielding can be achieved irrespective of whether the conductor is housed inside the cavity of the electrode.

If the opening of the cavity is covered by a circuit board, the conductive element may be a track on the circuit board. This simplifies the assembly of the signal acquisition device, wherein a circuit board is also present.

A second aspect of the invention provides a wearable patch for monitoring vital signs of a subject, the wearable patch comprising the signal acquisition device according to any of the embodiments of the first aspect of the invention. It is to be understood that the advantages described in the first aspect of the invention are also attainable by the second aspect of the invention.

These and other aspects of the invention will be apparent from and elucidated with reference to the embodiments described hereinafter.

### BRIEF DESCRIPTION OF THE DRAWINGS

Figs. 1a and 1b show a known implementation of a signal acquisition device.
Figs. 2a and 2b show an example of a signal acquisition device according to the invention.
Fig. 3 shows another example of a signal acquisition device according to the invention.
Figs. 4a and 4b show another example of a signal acquisition device according to the invention.
Fig. 5 shows an example of a wearable patch for monitoring vital signs of a subject according to the invention.
Figs. 6a and 6b show another example of a wearable patch for monitoring vital signs of a subject according to the invention.

### DETAILED DESCRIPTION OF EMBODIMENTS

The invention will be described with reference to the figures. The detailed description and specific examples, while indicating exemplary embodiments of the invention, are intended for purposes of illustration only and are not intended to limit the scope of the invention. These and other features, aspects, and advantages of the present invention will become better understood from the following description, appended claims, and accompanying drawings. The Figures are merely schematic and are not drawn to scale. It should also be understood that the same reference numerals are used throughout the Figures to indicate the same or similar parts.

The invention provides a signal acquisition device. The signal acquisition device comprises an electrode for acquiring a signal from a subject and an amplifier configured to amplify or buffer the signal from the electrode to produce an amplified or buffered signal at an output of the signal acquisition device. The amplifier may amplify the signal, i.e., it can increase the amplitude of a signal which may be a time-varying voltage or current, or it may buffer the signal, by providing electrical impedance transformation, with the aim of preventing the signal source from being affected by interference. The amplifier may be a pre-amplifier, whose amplified signal can be transmitted to a second amplifier. The amplifier may be for example an operational amplifier. The signal acquisition device may acquire a signal from a subject, such as an electrocardiogram, an electrooculogram, an electromyogram, an electroencephalogram and an electrical-cardiotocogram signal.

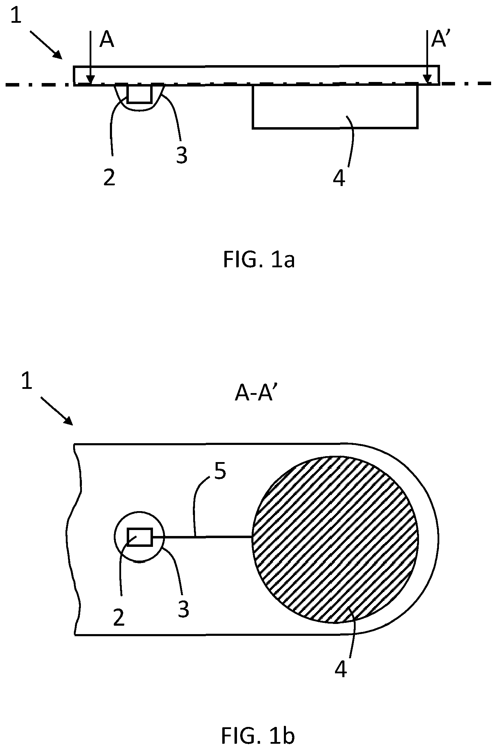

Figs. 1a and 1b show a known solution for a signal acquisition device 1 comprising an electrode 4 and an amplifier 2. The amplifier is mechanically shielded by a shield 3 to avoid its damage from mechanical loads and to avoid skin irritation or direct electrical contact with the skin. Shield 3 may be a casing or potting compounds e.g., based on epoxy. Conductor 5 is the wire or track connecting the output of the electrode 4 with the input of the amplifier 3. Conductor 5 is sensitive to electromagnetic interference, thus it needs to be shielded, to maintain the desired signal quality, expressed for example by a metric such as signal to noise ratio. The longer the distance between the amplifier 3 and the electrode 4, the longer conductor 5 is and the more the signal degrades due to electromagnetic interference.

The invention proposes to use the electrode itself for protection of the amplifier from mechanical and electrostatic loads. Another advantage is that the length of the conductor is minimized, so that not only interference but also degradation of signal quality due to other reasons, such as resistance, capacitance, and conductance per unit length of the conductor is limited. Embodiments may also provide electromagnetic interference shielding of the conductor connecting the electrode with the amplifier. The invention proposes that the electrode used for acquiring the signals of the subject comprises a cavity, which cavity has the required dimensions to house the amplifier. The cavity of the electrode may for example have a diameter of up to 10 mm, but a greater diameter may be used according to the dimensions of the used amplifier and/or electrode. The thickness of the electrode in its part that does not comprise the cavity, may be the thickness of commonly used electrodes, but it may also be thicker, e.g., up to 3 mm thicker. A thicker electrode may permit a better skin contact. Nevertheless, when applied on the skin, the part of the electrode that is not in contact with the skin is susceptible to electromagnetic interference. Thus, the thickness of the electrode is preferably chosen in such a manner, that when the electrode is positioned on a user, the part of the electrode not in contact with the skin is minimized.

The gist of the invention is that it is the electrode itself that comprises the cavity where the amplifier is housed. This is different to a signal acquisition device, which comprises an enclosure, an electrode, an amplifier and possibly other components, wherein the enclosure and not the electrode has a cavity for housing the amplifier. An advantage of having a cavity within an electrode itself, rather than the cavity being in the enclosure of the signal acquisition device, is that complexity of the signal acquisition device and dimensions can be reduced, as an enclosure is not required. Furthermore, a signal acquisition device that is more robust, using less materials and potentially cheaper to construct may be achieved.

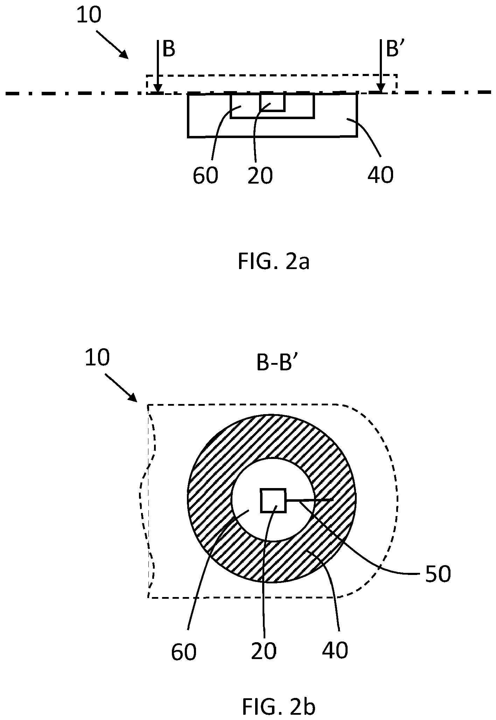

Figs. 2a and 2b show an embodiment according to the invention. A signal acquisition device 10 comprises an electrode 40 for acquiring a signal from a subject and an amplifier 20 configured to amplify the signal acquired by electrode 40. Electrode 40 comprises a cavity 60 wherein the amplifier 20 is housed. The cavity 60 in Fig. 2a is shown to have an opening opposite to the part of the electrode configured to be in contact with the subject or the subject's clothes, nevertheless, the cavity may have an opening in any part of the electrode, including the part configured to be in contact with the subject.

Fig. 2b is a rotated view of Fig 2a. In Fig. 2b, a conductor 50 is shown, which connects the electrode 40 to the amplifier 20. The conductor 50 delivers the signal acquired by electrode 40 to amplifier 20.

The conductor 50 may at least be partially housed within the electrode, which thus provides protection from electromagnetic interference. As the amplifier is also housed within the electrode, the conductor length is kept to a minimum.

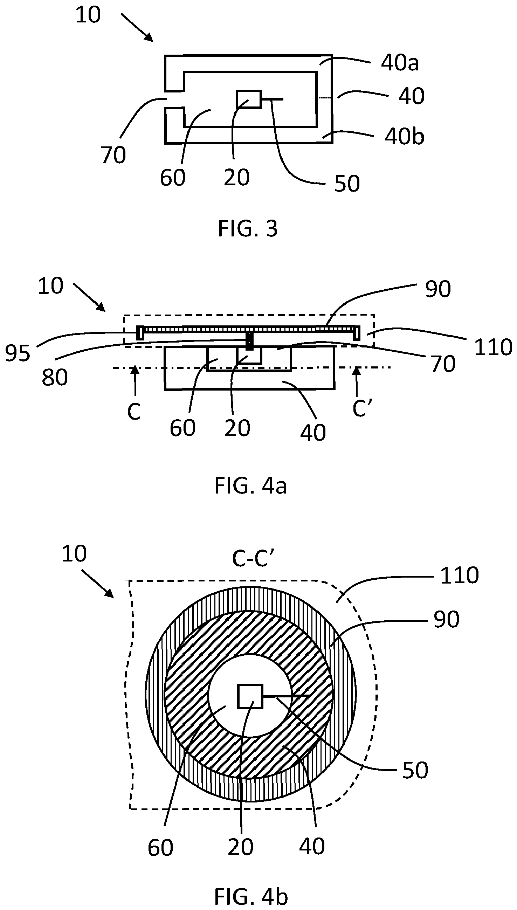

The signal acquisition device may be configured to be mounted on a substrate, e.g. a circuit board such as a PCB or a flexible circuit, as shown with dashed lines for example in Figs. 2a, 2b, 4a and 4b.

Fig. 3 shows another embodiment of the signal acquisition device 10. In this embodiment the electrode 40 substantially surrounds the amplifier 20. The cavity 60 may have an opening 70 for the output of the amplifier 20 to exit the cavity 60 of electrode 40. Alternatively, or additionally, the opening 70 may enable a part of a circuit board to have access to cavity 60. To implement this embodiment, the electrode may comprise 2 parts, 40a and 40b, which when assembled form electrode 40.

Figs. 4a and 4b show another embodiment of the signal acquisition device 10. As also shown in Figs 2a and 2b, the signal acquisition device 10 comprises an electrode 40 and an amplifier 20 configured to amplify the signal acquired by electrode 40 and carried by conductor 50 from the electrode 40 to the amplifier 20. Electrode 40 comprises a cavity 60 wherein the amplifier 20 is housed. The cavity 60 is shown to have an opening 70 opposite to the part of the electrode configured to be in contact with the subject or the subject's clothes, nevertheless, the cavity may have an opening in any part of the electrode, including the part configured to be in contact with the subject.

In this embodiment, a track 80 is connected to the output of the amplifier 20 and is configured to carry the amplified signal of the amplifier 20. The track 80 is also connected to a conductive element 90. Alternatively, the conductive element 90 may be directly connected to the output of the amplifier 20.

Alternatively, although not shown in Fig. 4a, the conductive element 90 may not be connected to the output of the amplifier, but it may carry a controlled voltage from another source, e.g., it may be connected to ground, or a power source. Preferably, the voltage source has a low output impedance, and the voltage has minimal fluctuations.

The conductive element 90 is positioned across an opening 70 of the cavity 60. In Fig. 4b, the conductive element 90 is exemplary shown to be disc shaped fully covering opening 70. Nevertheless, the conductive element 90 may have any shape, regular or irregular and it may partially cover the electrode 40 or an opening 70 of the cavity 60. For example, the conductive element 90 may comprise a peripheral element 95, positioned in the periphery of conductive element 90 as shown in Fig. 4a. Purpose of peripheral element 95 is to provide electromagnetic interference protection from a wider range of angles.

Conductive element 90, at least partially covers conductor 50. Thus, conductor 50 connecting the electrode with an input of the amplifier is at least partially shielded from interference. As a result, the conductive element 90 forms an interference shield configured to shield the conductor 50 from electromagnetic interference.

In case the conductive element 90 carries a controlled voltage having minimal fluctuations, from a low-impedance source, e.g., 0 V or voltage of a power source, passive shielding of the conductor 50 is achieved. A voltage with minimal fluctuations may only cause minimal dynamic distortion. However, a significant voltage difference can occur between the conductive element 90 acting as a shield and the electrode 40. As already explained, if the distance between the shield i.e., conductive element 90 and electrode 40 varies, e.g. due to mechanical stress, the shifting of electrical loads could cause disturbances, due to the microphonic effect. An advantage of the proposed signal acquisition device 10 comprising a conductive element 90, is that due to the rigidness of the signal acquisition device 10, the distance of conductive element 90 from the electrode 40, remains unchanged or changes minimally, in case the signal acquisition device is used, or in case it is under mechanical loads. As a result, electrical noise caused by a microphonic effect is also minimized.

In case the conductive element 90 is connected to the output of the amplifier 20, it carries a controlled voltage, which is the amplified or buffered signal, and active shielding of conductor 50 is thus achieved. In this case there is no significant voltage difference between the conductive element 90 acting as a shield and the electrode 40 and therefore no disturbances causing electrical noise.

The purpose of the conductive element 90 is thus to strengthen the protection of conductor 50 from interference as already described with regard to Figs. 2a and 2b. It is not necessary that the conductive element 90 is in contact with the electrode 40. In an embodiment the conductive element 90 is included in a circuit board 110 and the electrode 40 is mounted on the circuit board 110 in such a way, that the conductive element 90 is positioned across an opening 70 of the cavity 60 of electrode 40.

In a second aspect of the invention, a wearable patch 100 comprises the signal acquisition device 10 as has already been described herein. The wearable patch can be used for monitoring biometric parameters of a subject, e.g. based on an electrocardiogram, electrooculogram, electromyogram, electroencephalogram or an electrical-cardiotocogram.

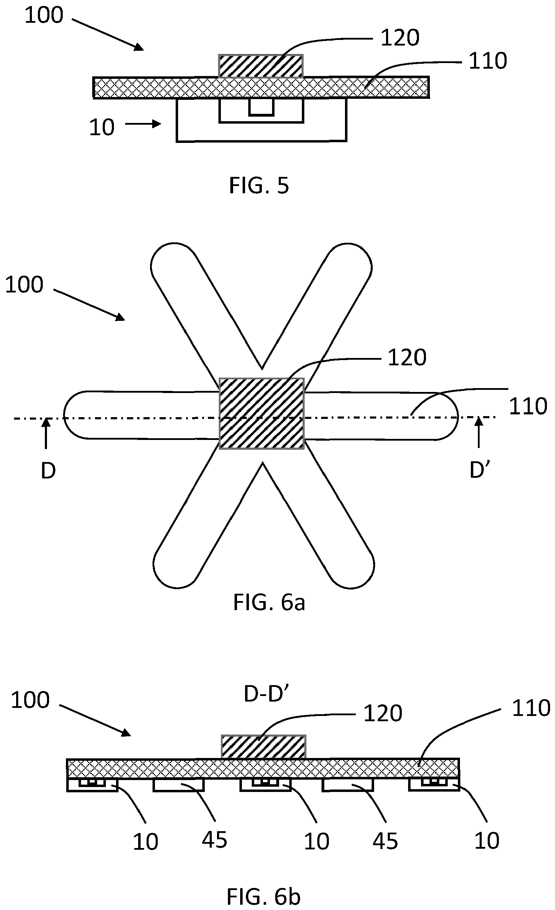

Fig. 5 shows an example of such a wearable patch 100 comprising a signal acquisition device 10. The wearable patch 100 may comprise a circuit board 110, e.g. a PCB or a flexible circuit. The wearable patch 100 may also comprise an electronics module 120, having all necessary submodules, enabling it to receive the amplified signal from the signal acquisition device 10. The electronics module may comprise any of a power source, a connection to a power source, a transmitter, a receiver, a CPU, a controller, a memory, and an amplifier. In an example, the electronics module 120 receives the output of the signal acquisition device which is the amplified signal acquired by electrode 40. The amplified signal may be amplified by a second amplifier of the electronics module 120. The electronics module may process the signal and transmit the processed signal to an external device like a monitor, a database or a monitoring device such as a patient monitor. The electronics module may alternatively not process the signal and it may directly transmit it to another device to be stored or processed.

Figs. 6a and 6b show another example of a wearable patch 100 comprising a plurality of signal acquisition devices 10. In the example shown in Fig. 6b, the wearable patch 100, may comprise electrodes 45 which do not house an amplifier 20. Electrodes 45 may be electrodes as already known in the state of the art. They may be used as ground electrodes, for common mode rejection, preventing power line noise from interfering with the biopotential signals of interest.

In summary, the invention provides a signal acquisition device 10 comprising an electrode 40 for acquiring a signal from a subject, and an amplifier 20 being configured to amplify or buffer the signal from the electrode, wherein the signal acquisition device comprises a cavity 60 housing the amplifier. In accordance with the invention, the electrode comprises the cavity housing the amplifier. The cavity within the electrode provides mechanical and electrostatic shielding to the amplifier.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. Signal acquisition device (10) comprising:
an electrode (40) for acquiring a signal from a subject, and
an amplifier (20) configured to amplify or buffer the signal from the electrode to produce an output signal at an output of the signal acquisition device, wherein the signal acquisition device comprises a cavity (60) housing the amplifier, **characterized in that**
the electrode comprises the cavity housing the amplifier.

2. Signal acquisition device according to claim 1, comprising:
a conductor (50) configured for connecting the output of the electrode with an input of the amplifier, wherein the electrode at least partially houses the conductor.

3. Signal acquisition device according to claims 1 or 2, further comprising:
a conductive element (90) configured for carrying a controlled voltage, wherein the conductive element is positioned across an opening (70) of the cavity.

4. Signal acquisition device according to claim 3, wherein the opening of the cavity is covered by a circuit board (110), the conductive element being a track on the circuit board.

5. A wearable patch (100) for monitoring biometric parameters of a subject, the wearable patch comprising the signal acquisition device (10) according to any of the preceding claims.
